# EUROPEAN PATENT SPECIFICATION

(11) **EP 2 883 958 B1**
(45) Date of publication and mention of the grant of the patent: **11.04.2018**
(21) Application number: 15150341.4
(22) Date of filing: 24.02.2010
(51) Int. Cl.: C12P 7/40, C12P 7/64

(54) **Improved methods for fermentative production of docosahexaenoic acid**
Verbesserte Verfahren zur fermentativen Herstellung von Docosahexaensäure
Procédés perfectionnés de production par fermentation d'acide docosahexaénoïque

(30) Priority: 25.02.2009 IN MU04282009
(43) Date of publication of application: 17.06.2015
(62) Divisional of application: 10745894.5
(73) Proprietor: V.B.Medicare Pvt. Ltd., Mumbai - 400 001, Maharashtra (IN)
(72) Inventor: Ratnam, Rakesh, Bangalore 560 048 Karnataka (IN); Aurora, Sundeep, Bangalore 560 048 Karnataka (IN); Talluri, Surendra, Bangalore 560 048 Karnataka (IN); Ganesan, Ellappan, Bangalore 560 048 Karnataka (IN); Peter, Vineetha, Bangalore 560 048 Karnataka (IN)
(74) Representative: Kuhnen & Wacker Patent- und Rechtsanwaltsbüro PartG mbB

(56) References cited:
- EP-A1- 0 823 475
- WO-A1-94/08467
- WO-A2-2007/069078
- US-A1- 2006 286 649
- US-A1- 2010 239 533
- ANITA N JAKOBSEN ET AL: "Accumulation of docosahexaenoic acid-rich lipid in thraustochytrid Aurantiochytrium sp. strain T66: effects of N and P starvation and O2 limitation", APPLIED MICROBIOLOGY AND BIOTECHNOLOGY, vol. 80, no. 2, 17 June 2008 (2008-06-17), pages 297-306, XP019623690, ISSN: 1432-0614
- ANITA N JAKOBSEN ET AL: "Endogenously Synthesized (-)-proto-Quercitol and Glycine Betaine Are Principal Compatible Solutes of Schizochytrium sp. Strain S8 (ATCC 20889) and Three New Isolates of Phylogenetically Related Thraustochytrids", APPLIED AND ENVIRONMENTAL MICROBIOLOGY, vol. 73, no. 18, 1 September 2007 (2007-09-01), pages 5848-5856, XP008153839, ISSN: 0099-2240, DOI: 10.1128/AEM.00610-07 [retrieved on 2007-07-27]
- SONG ET AL: "Optimization of fermentation parameters for the biomass and DHA production of Schizochytrium limacinum OUC88 using response surface methodology", PROCESS BIOCHEMISTRY, vol. 42, no. 10, 15 September 2007 (2007-09-15), pages 1391-1397, XP022250160, ISSN: 1359-5113, DOI: 10.1016/J.PROCBIO.2007.07.014
- SHWU-TZY WU ET AL: "Production of Docosahexaenoic acid and succinic acid by schizochytrium sp. S31", FOOD SCIENCE AND AGRICULTURAL CHEMISTRY, vol. 4, no. 2, 1 June 2002 (2002-06-01), pages 62-66, XP008153840, ISSN: 1560-4152
- ZHANYOU CHI ET AL: "Study of a two-stage growth of DHA-producing marine algae Schizochytrium limacinum SR21 with shifting dissolved oxygen level", APPLIED MICROBIOLOGY AND BIOTECHNOLOGY, vol. 81, no. 6, 21 October 2008 (2008-10-21), pages 1141-1148, XP019654222, ISSN: 1432-0614
- CHANG GUIFANG ET AL: "Improvement of docosahexaenoic acid production on glycerol bySchizochytriumsp. S31 with constantly high oxygen transfer coefficient", BIORESOURCE TECHNOLOGY, vol. 142, 6 May 2013 (2013-05-06), pages 400-406, XP028576165, ISSN: 0960-8524, DOI: 10.1016/J.BIORTECH.2013.04.107

## Description

### TECHNICAL FIELD

Invention relates to improved methods for fermentative production of docosahexaenoic acid. More particularly it relates to new media compositions containing soybean meal and other nutrients for enhanced production of fats, more particularly, polyunsaturated fatty acids by batch fermentation using Thraustochytriales in liquid media. The invention also relates to new methods of fermentation of docosahexaenoic acid.

### BACKGROUND OF INVENTION

Essential fatty acids are those which are essential in the health of an organism but can not be synthesized in animal / human body and must be supplied through food sources. Omega-6 fatty acids and Omega-3-fatty acids are the fatty acids amongst this group that play a crucial role in brain function as well as normal growth and development. Both these classes of essential fatty acids belong to polyunsaturated fatty acids (PUFAs), generally necessary for stimulating skin and hair growth, maintaining bone health, regulating metabolism, and maintaining reproductive capability.

A family of polyunsaturated fatty acids that have a carbon-carbon double bond in the ω-3 position are known as Omega 3 fatty acids. α-linolenic acid, eicosapentaenoic acid, and docosahexaenoic acid, that have have either 3, 5 or 6 double bonds in *cis*-configuration in a carbon chain of 18, 20 or 22 carbon atoms respectively, are considered as Omega 3 fatty acids important in human nutrition.

Amongst Omega-3 fatty acids, Long Chain-PUFAs, also abbreviated as LC-PUFAs, by definition, are fatty acids that have chain lengths greater than 18 carbon atoms and contain two or more double bonds. One of the Fatty acids that is important in infant nutrition is docosahexaenoic acid (DHA). This fatty acid is present in small concentrations in human milk.

Both term and pre-term infants can synthesize the LC-PUFAs from the respective essential fatty acids, but controversy has centered around the fact that breastfed infants have higher plasma concentrations of these DHA than formula-fed infants. This information has been interpreted to imply that formula-fed infants cannot synthesize enough DHA to meet ongoing needs (In "A shore-based diet rich in energy and 'brain-specific' nutrients made human brain evolution possible": Proceedings of the Ghent Conference on Water and Human Evolution by Stephen C. Cunnane Department of Nutritional Sciences, University of Toronto, in http://users.ugent.be/∼mvaneech/Cunnane.html)

The US National Institutes of Health published Recommended Daily Intakes of fatty acids. According to which EPA and DHA are recommended 650 mg/day, Alpha linolenic acid is recommended 2.22 g / day and linolenic acid is recommended in 4.44 g per day and saturated fats are recommended not to exceed 8%of total calorie intake or about 18 g/day.

The only feasible conventional source of EPA and DHA is fish, that too only certain kind of fish that are known as "oily fish". To fulfill recommended daily need of DHA, recommendation of American Heart Association (http://www.americanheart.org/presenter.jhtml?identifier=4632) has released a science advisory: "We recommend eating fish (particularly fatty fish) at least two times a week. ----------------- Fatty fish like mackerel, lake trout, herring, sardines, albacore tuna and salmon are high in two kinds of omega-3 fatty acids, eicosapentaenoic acid (EPA) and docosahexaenoic acid (DHA). --------- We also recommend eating tofu and other forms of soybeans, canola, walnut and flaxseed, and their oils. These contain alpha-linolenic acid (LNA), which can become omega-3 fatty acid in the body. The extent of this modification is modest and controversial, however. More studies are needed to show a cause-and-effect relationship between alpha-linolenic acid and heart disease.." However, that is hardly a feasible source to fulfill the potential requirements because demand is higher than that can be supplied through catch of oily fish. Very few Americans can get for themselves this quota for two main reasons, availability as well as cost. Hence, from ecological sustainability point of view, fish is not a feasible source of supply of dietary DHA. Furher, fish is known to contain high quantity of mercury, and this also puts a limitation on its intake and keeps it forbidden for children and pregnant women. Still further, with DHA, fish also has about six times higher amount of omega 6 fatty acids which counteract its therapeutic effect/s.

In view of inadequacy of conventional sources of DHA, marine protists have been focussed upon as alternative source of DHA by their heterotropic production. Hence, improvement in efficiency of heterotropic production of these organisms for production of DHA is highly desirable.

### PRIOR ART

US5340742 has claimed a process for growing Thraustochytrium, Schizochytrium, and mixtures thereof, in a culture medium containing less than about 3 grams of chloride per liter of said culture medium, sources of carbon, nitrogen, micronutrients, and a non-chloride sodium salt at a temperature from about 5 degree C. to about 48 degree C. and at a pH from about pH 5.0 to about pH 11.0.

Kyle et al in US5407957 describes a process in which a single cell edible oil containing at least about 20% docosahexaenoic acid (DHA) in triglyceride form comprises cultivating heterotrophic microalgae of the class Dinophyceae capable of producing said single cell oil in an aerated fermenter containing a nutrient solution having a limiting nitrogen source and an oxygen level of at least about 10% of air saturation level and continuing cultivation to achieve a cell density of at least about 10 grams biomass per liter of nutrient solution.

Kumar et al describe, in US 6410282, a method for enhancing levels of docosahexaenoic acid and eicosapentaenoic acid in Thraustochytrid fungi, by culturing the Thraustochytrid fungal strain *Ulkenia radiata* Gaertner, deposited at the National Institute of Bioscience & Human Technology, Japan and bearing Accession No. AB22115, in a culture medium for 2 to 5 days at 25 degree C. to 30 degree C.; inoculating a medium containing 0.1 to 1% polyvinyl pyrrolidone with the Thraustochytrid fungal strain for 3 days at 25 degree C. to 30 degree C.; and harvesting the cells by centrifugation and extracting the enhanced amounts of docosahexaenoic acid and eicosapentaenoic acids from the cells.

US6451567 describes a process for producing lipids by growing euryhaline microorganisms in a fermentation medium, wherein said euryhaline microorganisms are capable of producing about 1.08 grams of long chain omega-3 fatty acids per liter of the fermentation medium per day. The medium consisting of 40 grams of sugar per liter of the fermentation medium at a sodium ion concentration in the fermentation medium of 60% seawater. Further the process claims that the euryhaline microorganisms have exponential growth rates of at least about 5 doublings per day and 7 doublings per day at 25 degree C and 30 degree C respectively.

Barclay in US6566123 reported biomass comprising microflora selected from the order Thraustochytriales, wherein said microflora is produced by a process comprising growing said microflora at a temperature from about 5 degree C to about 48 degree C in a culture medium containing less than about 3 grams of chloride per liter of said culture medium; sources of carbon, nitrogen, and nutrients; and a non-chloride sodium salt.

Bailey et al in US6607900 described a fed batch fermentation process for producing lipids containing polyenoic fatty acids from microorganisms of the order Thraustochytriales that produce at least about 20% of their dry cell weight as lipids, comprising adding to a fermentation medium comprising said microorganisms a non-alcoholic carbon source and a nitrogen source at a rate sufficient to increase the biomass density of said fermentation medium to at least about 100 g dry cell weight per liter, wherein said process comprises a biomass density increasing stage and a lipid production stage and wherein the level of dissolved oxygen present in said fermentation medium during said biomass density increasing stage is at least about 4% of saturation in said fermentation medium and wherein the level of dissolved oxygen present in said fermentation medium during said production stage is less than about 3% of saturation in said fermentation medium.

A process for producing lipids comprising: (a) growing microorganisms of the order Thraustochytriales in a fermentation medium; and (b) extracting lipids from said microorganisms, wherein said lipids have at least 94.0% by weight of total omega-3 fatty acids as C22:6n-3 (DHA) is reported by Barclay in US 7005280.

US7011962 describes a method for producing lipids by growing microorganisms of the order Thraustochytriales in a fermentation medium; and extracting lipids from said microorganisms wherein at least about 49% of the total fatty acids of said lipids are omega-3 fatty acids; wherein said microorganisms have exponential growth rates of at least about 5.5 doublings per day when grown under the following conditions: exponential phase of growth in M-5 media at 25 degree C. in a flask on an orbital shaker. The growth conditions are achieved by inoculating a 250 ml flask having 50 ml of M-5 media adjusted to pH 7.0 with 12 ml of a culture having said microorganisms in exponential growth phase, and shaking said flask at 200 rpm on an orbital shaker for 22.75 hours

Behrens in US7163811 describes a method of producing docosahexaenoic acid (DHA) by culturing heterotrophic microalgae of the class Dinophyceae in a culture medium, wherein the medium comprises: (a) chloride ion at a concentration of less than or equal to about 2 g/l; and (b) potassium ion at a concentration of greater than or equal to about 0.25 g/L; wherein the microalgae produces at least about 0.04 g DHA per 10⁹ cells.

US7022512 claims an isolated microorganism selected from the group consisting of: a) Schizochytrium ATCC No. 20888 or a strain derived therefrom; b) Schizochytrium ATCC No. 20889 or a strain derived therefrom; c) Thraustochytrium ATCC No. 20890 or a strain derived therefrom; d) Thraustochytrium ATCC No. 20891 or a strain derived therefrom; and e) Thraustochytrium ATCC No. 20892 or a strain derived therefrom. Thraustochytrium, Schizochytrium, and mixtures thereof are grown in fermentation medium containing non-chloride containing sodium salts, in particular sodium sulfate and produces microflora having a cell aggregate size useful for the production of food products for use in aquaculture and a food product which includes Thraustochytrium, Schizochytrium, and mixtures thereof, and a component selected from flaxseed, rapeseed, soybean and avocado meal. Such a food product includes a balance of long chain and short chain omega-3 highly unsaturated fatty acids.

US 20060286649 pointed out that cost of producing microbial lipids containing polyenoic fatty acids, and especially the highly unsaturated fatty acids, such as C18:4n-3, C20:4n-6, C20:5n3, C22:5n-3, C22:5n-6 and C22:6n-3, have remained high in part due to the limited densities to which the high polyenoic fatty acid containing eukaryotic microbes have been grown. It has also been pointed out in US 20060286649 that attempts to get high densities in batch fermentations are saddled with several difficulties. The limited oxygen availability both at these high cell concentrations and the higher temperatures needed to achieve high productivity are one of the limitations. Therefore, a need is acknowledged for a process for growing microorganisms at high concentration which still facilitates increased production of lipids containing polyenoic fatty acids. It was further pointed out that very high density cultivation (greater than about 100 g/L microbial biomass, especially at commercial scale), instead of resulting in high yields of polyunsaturated fatty acid, can actually lead to decreased polyenoic fatty acid contents and hence decreased polyenoic fatty acid productivity, due in part to several factors including the difficulty of maintaining high dissolved oxygen levels due to the high oxygen demand developed by the high concentration of microbes in the fermentation broth. It is pointed out that methods to maintain higher dissolved oxygen level include increasing the aeration rate and/or using pure oxygen instead of air for aeration and/or increasing the agitation rate in the fermentor, however, it was also pointed out that, they can cause additional problems including severe foaming problems in the fermentor, microbial cell breakage leading to the lipids to be released in the fermentation broth where they can become oxidized and/or degraded by enzymes, ultimately resulting into lipids that generally contain only very small amounts of polyenoic fatty acids. Data provided by them in Fig 1 reveals the productivity of DHA obtainable under various contents of dissolved oxygen levels between 5 to 40% level that ranged between 11.3 % for 5% Dissolved Oxygen level to 5.6 for 40% dissolved oxygen level. Corresponding figures for DHA g/L are 2.0 to 1.0. Hence, US 20060286649 discouraged use of oxygen bubbling and agitation as methods of improving efficiency of fermentation. Further, it is also acknowledged that (para 0029) when the substrate is added to the batch fermentation process the large amount of carbon source present (e.g. more than 200 g/L or more per 60 g/L of biomass density) had a detrimental effect on the microorganisms. To circumvent all these problems, US 20060286649 disclosed a new method to produce polyenoic fatty acids comprising achieving high cell densities of at least 100 g/ L of dry cells by a fed batch process to increase biomass density, wherein nutrients are added in small increments and it was found that in this process there is no need of elevating dissolved oxygen concentration for achieving high cell densities, and then switching to a lipid production phase by actually reducing the oxygen concentration, preferably to zero percent led to increase of production of polyenoic fatty acids. Limitations of a batch process were also evident from Example 5 of US 20060286649 which concluded that in general, extra nitrogen has a negative effect on fermentative performance, as significant reductions were observed in the DHA productivity, and since in absence of enough nitrogen there will be a limitation to the carbon that can be utilized, the batch process has limitations in DHA productivity. Thus, US 20060286649 was conclusive in ruling out batch process as viable option for high density cultivation and for improving productivity of DHA production system utilizing Thraustochytriales. They invented that Fed batch process allows addition of incremental quantities of nutrient without the need of very high levels of dissolved oxygen levels. During the phase of increase in biomass density, oxygen concentration of about 8% was reported to have been maintained by controlling the amount of oxygen in the headspace and that was enough to support production of dry biomass density of about 100 g/L or more by controlling the speed at which the fermentation medium is stirred enough to support cell growth in fed batch mode, but cell breakage is avoided. Dissolved oxygen of more than 8% was specifically avoided to avoid adverse effects noted above that lead to lower yield of DHA.

Jakobsen et al. (2008) "Accumulation of docosahexaenoic acid-rich lipid in thraustochytrid Aurantiochytrium sp. strain T66: effects of N and P starvation and 02 limitation", Appl. Microb. Biotech. 80(2):297-306, and Jakobsen et al. (2007) "Endogenously Synthesized (-)-proto- Quercitol and Glycine Betaine Are Principal Compatible Solutes of Schizochytrium sp. Strain S8 (ATCC 20889) and Three New Isolates of Phylogenetically Related Thraustochytrids", Appl. Environ. Microbiol. 73(18):5848-5856, disclose processes of producing DHA from the thraustochytrid *Aurantiochytrium* sp. strain T66 in submerged fermentation with maintaing dissolved oxygen at more than 8% using stirring and aeration.

However, a fed batch process is cumbersome in actual practice to optimize for productivity and for control on contamination. Batch process is easier to control and optimize, involving only one time operation with respect to composition of the medium to be fermented and inoculation. Hence, there is a need to as much as possible overcome the known limitations of a batch process. Further, there is also a need to explore alternative methods of fermentation.

In particular, the objects are achieved by a process according to claim 1. Preferred embodiments are subject of dependent claims.

### SUMMARY OF INVENTION

This invention embodies novel processes of heterotropic fermentation by cultivating *Thraustochytriales.*

The invention comprises a process of producing polyunsaturated fatty acids from Thraustochytriales at high availability of oxygen which is done by maintaining dissolved oxygen in a submerged fermentation at more than 8% saturation throughout the fermentation that resulted in obtaining more than 2 g DHA per liter of fermentation medium; wherein the process comprises steps of initially increasing the agitation tip speed of a stirrer used to stir the medium up to a tip speed of 3.2 m/sec, and of switching to gas mix mode where pure oxygen is mixed with air and sparged through the fermenter medium to prevent dissolved oxygen level dropping below a targeted level of around 15% or 20% or 30% or 50% of saturation or more in an advanced stage of fermentation in addition to agitation at said tip speed. In one embodiment of submerged fermentation, the invention may comprise a batch fermentation, a semicontinuous fermentation or a continuous fermentation. Also disclosed herein is a process comprising a batch fermentation wherein by maintaining at least 20% dissolved oxygen level in the medium, yields higher than normally obtained in prior art conventinal batch fermentation are obtained i.e. a yield of 1.3 g/Liter of DHA, more preferably higher than 3 g/Liter, still more preferably higher than 10g/Liter are obtained. Further disclosed herein is a process comprising maintaining at least 20% dissolved oxygen level by using comprising one or more means comprising (a) sparging of air mixed with oxygen through the medium, (b) by stirring the medium at 800 rpm speed that may comprise increasing the agitation tip speed up to 3.2 m/ sec, (c) switching to gas mix mode where pure oxygen is mixed with air and sparged through the fermenter medium to maintain the dissolved oxygen levels as and when required to prevent dissolved oxygen level dropping below targeted level or for maintaining oxygen saturation of around 50% or more. Using above measures of increasing dissolved oxygen level separately or in combination do not cause adverse effects on the cells and help in maintaining high cell density without cell breakage. Further disclosed herein is a process of obtaining a biomass density of more than 100g/L dry cell weight with DHA accumulation of 2 g/Liter or more' preferably to more than 10 g/Lit. Further disclosed herein is that for achieving high productivity of DHA production, the oxygen to air ratio that is sparged is varied from 1% to 60% oxygen. The word "sparged" has been used in this specification with a meaning that it is pumped in the liquid medium in the fermenter under pressure generating a massive and forceful stream of tiny bubbles resulting in good contact with the air that is bubbled with the liquid medium and also results in good agitation. Further disclosed is a process comprising a batch process further comprising adding reducing sugars up to 450 g/Liter to the fermentation period at the beginning of the batch fermentation. Further disclosed herein is a process comprising a batch process wherein inorganic or urea nitrogen addition is supplemented with addition of organic nitrogen derived from amino groups containing compounds. The said organic nitrogen containing peptide bonds may be a protein rich source, a peptide rich source or amino acids. The said protein rich source may comprise defatted oil-cakes comprising defatted soy-flour. Further disclosed herein is a process which comprises assisting addition of anti-foaming agent, including food grade silicone antifoam agent. Further disclosed herein is a process which may be a semi-continuous process comprising steps of (a) fermenting a batch upto a point where the organism is in actively dividing state in a log phase of growth in first fermenter, (b) retaining a portion of the fermenting medium in the first fermenter in a volume enough to provide an actively dividing inoculum to the next batch, aseptically transferring the rest of the actively fermenting medium to a second reactor, (c) restoring the volume of the first reactor by adding sterile fermentation medium to the same and subjecting the same to the step (a) and repeating this step for desired number of times, or optionally (i) to allow the balance volume to step (d) or (ii) to restore the volume of the first reactor by adding sterile fermentation medium to the same and allowing it to complete further growth and lipid production which is to be harvested to end the series of semi-continuous production, (d) providing conditions to the medium in the second fermenter to divide upto their potential that is limited by the nutrient availability and to produce DHA, (e) recovering the DHA . Further disclosed herein is a process comprising a continuous processs comprising the steps of (a) fermenting a batch upto a point where the organism is in actively dividing state in a log phase of growth in first fermenter,(b) taking precautions to avoid entry of contaminants, starting addition of sterile medium to the first fermenter and allowing aseptic drain off or removal of same volume of fermenting medium to a second fermenter at such a rate that the contents of the first reactor remain always at log phase of growth,(c) providing conditions to the medium in the second fermenter to divide upto their potential that is limited by the nutrient availability and to produce DHA, (d) after the second fermenter fills up to the capacity, provising for aseptic drain off or removal of medium excess of the capacity from the second fermenter, (e) recovering DHA immediately or after providing a further interval of time. Further disclosed herein is a process which comprises sparging of oxygen through the fermentation medium to maintain high content of dissolved oxygen. The oxygen may preferably be sparged mixed with air in a ratio of 1% to 60% depending on the requirement. Further disclosed herein is a process comprising a batch process that comprises adding all nutrients in the beginning and achieves a dry biomass density of at least about 100 g/L of fermentation medium, generally about 120 - 130g and a yield of DHA is at least about 10 g/L, generally about 12 - 15g per liter of the medium. Further disclosed herein is that the carbohydrate nutrients may be a fermentable monosaccharide all of which is added in the beginning of fermentation in a quantity enough to achieve a dry cell weight density of at least 100 g/L cell dry weight.

### DETAILS OF TNE INVENTION

The liquid state fermentation has been described as "submerged fermentation". In the process of this invention, means for providing high dissolved oxygen supply throughout the fermentation have been devised that would avoid the known disadvantages of maintaining high dissolved oxygen concentration, that relate to high cell breakage, depression in DHA synthesis in lipid production phase etc. and achieve DHA productivity better than known productivity for a conventional batch process operated at high dissolved oxygen concentrations.

Batch fermentation, being simplest and most preferred presently, has received further attention on optimization and results given below reflect so. The results given for the alternative models semi-continuous fermentation and continuous fermentation are results of only exploratory experiments and are reported because they do show a potential to give higher productivities than the conventional batch processes and since there is a lot of further scope for making optimization and improvements in them, they may show better productivities in course of time than indicated here. However, even in the present preliminary state of results, they perform better than the conventional batch processes and do show a promise as inventive methods having a high potential for improved productivity of DHA by fermentation.

For the purpose of comparing the DHA productivities of the disclosed methods, the productivities of prior art conventional methods of batch cultivation with respect to yield of DHA g/Liter at various dissolved oxygen concentrations as disclosed in data given in Figure 1 in US 2006/0286649 are pertinent. That data is given for *Schizochytrium* ATCC 20888 wherein a productivity of 2.0 to 1.0 g/Liter is reported for DHA at dissolved oxygen ranging from 5 to 20 %. There would be differences in productivities amongst strains. However, this result would be useful to compare productivities of the same strain amongst different methods as an absolutely valid comparable standard and for other strains of *Schizochytrium* as at least fairly valid standard for comparison. On this basis, results given for 20% dissolved oxygen, a productivity of 1.3 g/Liter, can be taken as benchmark for assessing significance of productivities achieved for the process of this invention for a batch process at least for using dissolved oxygen % of 20% and higher and also for semi-continuous and continuous fermentation processes using dissolved oxygen levels around or above 20% dissolved oxygen concentration.

Contrary to the indications from the prior art knowledge, as discussed in details in the closest document US 2006/0286649 which resorted to only a moderate increase in dissolved oxygen concentration in biomass increase phase and decrease in oxygen availability in lipid production phase, of adverse impact of high concentration of reducing sugars, adverse impact of high nitrogen levels and adverse impact of measures available for improving dissolved oxygen levels on lipid and DHA yield arising form cell breakage, leakage and oxidation of lipids, and overall discard of the idea of improving availability of oxygen as a viable direction for improvement in productivity of DHA production by fermentation, it was surprising finding of this invention that sustained high levels of dissolved oxygen above 8 % upto 20% to 30% and in some cases even up to 50% could be devised and that appreciably high DHA productivity to about 10 g/Liter or higher could be achieved from fermentation using Thraustochytriales.

Most surprising is achievement of batch fermentation of more than 200g/L and up to 450 g/L of dextrose added right in the beginning of fermentation wherein the barrier of high concentration of reducing sugars could be overcome, all nitrogen necessary for its conversion could be added right in the beginning and the oxygen levels that start falling rapidly as cell density rises could be maintained at or above 20% upto 30 to 50% also without cell breakage. This is a result of combination of surprising finding that the organism could withstand high concentration of reducing sugars, combined with the finding that during fermentation of this high a quantity of reducing sugars per liter, its high requirement of dissolved oxygen could be provided without cell breakage by the combination of techniques found out by the inventors and high nitrogen requirement could also be satisfied through batch addition of organic nitrogen that is in amino form derived from proteins eukaryots that are not expensive too. In the instant case, defatted soy flour was used as nitrogen supplement with amino nitrogen for its high protein content, low cost and ready availability. In its place, it is possible to use any other nitrogen supplement with amino nitrogen such as peptides or amino acids that could preferably be available in low cost.

The importance of achievement of ability to ferment high concentrations of reducing sugars can be seen from the fact that on one hand they do not tolerate high concentrations of reducing sugars, a batch fermentation will require all the carbon sorce to be added right in the beginning and it was seen that these organisms do not utilize disaccharides or polysaccharides directly. The fermentation medium containing disaccharides when used for growth of either of the organisms namely *Schizochytrium limacinum* ATCC MYA 1381, *Schizochytrium aggregatum* ATCC 28209, *Thraustochytrid* PRA 148 yielded very poor quantities of lipids and DHA. Monosaccharides are one of the best carbon sources such as dextrose, fructose, etc and there seems to be no alternative to that. If complex polysaccharide sources are used such as rice grains, ragi, sorghum etc, to avoid the high concentration of reducing sugars in the beginning, enzymes capable of hydrolyzing them in course of time could be added leading to formation of simple sugars that are taken up by the organism resulting in high yields of DHA. However, the enzymes are expensive and the performances are easily reproducible.

It was found that maintaining dissolved oxygen level of 20-30% without cell breakage could be achieved initially by increasing the agitation tip speed up to 3.2 m/ sec. When in advanced stage of fermentation, the dissolved oxygen starts dropping below 30%, in addition to agitation at the said tip speed, the dissolved oxygen level could be restored without cell breakage and retaining appreciably high DHA productivity by switching the fermenter to gas mix mode where pure oxygen is mixed with air at a proportion and sparged through the fermenter medium. Air mixed with pure oxygen may carry 1 to 60% oxygen.

The increased agitation beyond 3.2m/sec of tip speed had detrimental effect on the organism due to cell lysis. The air flow under gas mixing could be increased up to 0.7 VVM, beyond 1 VVM resulted in cell lysis. The oxygen to air ratio was varied from 1% to 60% oxygen without any cell disruption. It is an embodiment of this invention that by appropriate combination of increased agitation, air flow under gas mixing and air to oxygen ratio, it is possible to maintain dissolved oxygen percentage during fermentation upto 30 to 50% without leading to cell breakage.

Productivity of DHA in g/Lit of medium varied with strains used, as can be seen from results of Experiments 5, 7 and 8. However, the evidence that increase in dissolved oxygen supply leading to substantial increase in productivity of DHA in terms of g/Liter can be seen from the results obtained for example no. 5 and 6 using *Schizochytrium* ATCC 20889. In example 5, dissolved oxygen concentration was maintained at 20% or above. Oxygen mix was used to maintain dissolved oxygen level at or above 20%. In Example no. 6, dissolved oxygen level was maintained at 20% by increasing aeration upto 800 rpm. However, dissolved oxygen level dropped below after a few hours in absence of oxygen bubbling to less than 5% from 48 hours till end of fermentation. Total dry biomass accumulation in Example 5 was 84 g/Liter, whereas in Example no. 6 the same was 44.8. Corresponding figures for DHA were 12 g/Liter for Experiment 5 and 4.2 for Experiment 6.

It is a further finding that the nitrogen requirement of a batch that exceeds the quantity of nitrogen that can be safely added through inorganic supplements, could be added in the form of organic compounds containing amino nitrogen.
Semi-continuous, continuous were explored as alternative processes for production of DHA at high oxygen concentration. All of them yielded satisfactory quantity of DHA in exploratory experiments and can be optimized to express their best potentials of DHA production wherever alternatives to batch process are desired.
High cell densities of around 100 g/Liter dry biomass density could also be achieved in semi-continuous fermentation by maintaining dissolved oxygen level to 30% or above.

The submerged fermentations of this disclosure involving high volume air-gas mixture sparging, the production of foam was tackled by adding anti-foam agents to the medium. Preferred antifoam agents are food grade anti-foams. Most preferred one is silicone antifoam. However, any other equivalent anti-foam agent can be used in its place.
In a semi continuous process of producing lipids from microorganisms by carrying a batch fermentation at a temperature of 20 to 30°C, pH 4.0 to 7.0, and above 50% oxygen saturation in first 24 hours and above 30% in next 48 hours with oxygen and gas mix arrangement with PID control , to produce a dry cell weight fermented biomass up to about 85g/L without allowing it to reach dry cell weight 100g/L dry cell weight of fermentation medium and draining off a part of the fermented medium accompanied by addition of fresh medium to replenish the batch and fermentation is continued under similar conditions to produce a dry cell weight fermented biomass and again part of the fermented mass is drained and is replenished again. This cycle was repeated. The semi-continuous method described above were also adapted to a process in which the biomass density of dry cell weight fermented medium reached more than 100g/L too.

The drained fermentation medium is collected in another bioreactor wherein either the lipids are extracted immediately from this batch or the lipid production is carried out at a temperature of 20 - 30°C without allowing the dry weight biomass density to exceed 100g/L over a period of time, pH 3.0 to 6.0 after which the lipids produced are recovered and the next lot of biomass for lipid production is taken up. It is also possible to allow the dry weight biomass density to reach to further increase as much as possible and then lipids are recovered. Productivities of 18 g DHA / liter were achieved in the exploratory experiments, which can be further improved by further optimization.

In yet another embodiment, a continuous process of producing lipids from microorganisms by carrying a batch fermentation at a temperature of 20 to 30°C, pH 4.0 to 7.0 at above 50% with pure oxygen mix with air in the first fermenter in first 48 hours, then continuously draining off fermented mass at a certain rate and also continuously replenishing the first fermenter with fresh medium at about same rate by fresh sterilized medium in such a way that the volume of fermentation medium in the fermenter is kept essentially constantand biomass density of the medium does not reach or exceed dry cell weight of 100 g/L. The drained fermentation medium is collected in second bioreactor wherein it is subjected to extraction of lipids or subjected to a further lipid production step at a temperature conducive for lipid production over a period of time , at pH 3.0 to 6.0 after which the lipids produced are recovered; and the process applied to the drained off medium repeated for next batch of drained medium. The continuous fermentation method described above can be adapted to a process in which the biomass density of fermented medium reached dry cell weight of more than 1 00g/L too.

The following examples are not to be construed as limiting the scope of invention but are to be construed as illustrative only in nature. Additional variations, including new media composition, new microbial strains or species of Thraustochytriales may be used in place of the Thraustochytriales and their strains used in these exploratory and illustrative experiments. Throughout the specification, wherever relevant, a mention of singular also includes plural of same kind; thus "a medium" includes one or more media performing same function.
Throughout the specification, expressions such as dry mass, dry matter, dry biomass are used that all indicate dry matter content.

### Example 1 - Fermentation with sucrose

### (A) Start of seed culture - Schizochytrium limacinum ATCC MYA 1381

100 ml of seed medium was prepared in 250 ml conical flask containing 100 ml water, 0.2 g dextrose, 2g of sucrose, 0.25 g yeast extract, 4 g soy flour and 3.2 g sea salt. The pH was adjusted to 7.5 using 1 N sodium hydroxide and was autoclaved. One loop full of Schizochitrium limacinum ATCC MYA 1381 was transferred to the flask and was incubated at 22°C at 220 RPM for 48 hours.

750 ml of seed medium was prepared in 1000 ml conical flask containing 750ml water, 1.5 g of dextrose, 15g of sucrose, 7 g starch, 2 g of Bacterial amylase, 1.125 g yeast extract, 30 g soy flour 1.125 g ammonium phosphate and 24 g sea salt. The pH was adjusted to 7.5 using 1 N sodium hydroxide and was autoclaved. 7.5 ml of seed inoculum generated from 100 ml seed flask was transferred to it. The flask was kept in a shaker for 58 hours at 24°C.

### (B) Submerged batch fermentation

5000 ml of water was taken in a 5L fermenter. 20 g of dextrose, 200 g of sucrose, 15 g of yeast extract, 35 g glycerol, 350 g soy flour, 14 g of ammonium phosphate and 160 g of sea salt was added to the fermenter and all the contents were dissolved. The pH of the contents in the fermenter was adjusted to 3.5 using dilute HCl. The sterilization of the media components was carried out and after that the pH was adjusted to 7.5 using 1 N sodium hydroxide. 500 ml of inoculum from example 1 was transferred via peristaltic pump.

The temperature was maintained at 22°C for 12 hours. The temperature was allowed to rise till 26°C and was not allowed to proceed further. The dissolved oxygen was not allowed to drop below 20% and the stirrer RPM was increased till 800 RPM. The pH of the medium was not allowed to drop below 5.0. Samples were taken intermittently and analyzed for biomass and the DHA content.

The fermentation was stopped after 120 hours and the following results were obtained. The total biomass accumulated was found to be 320 g wet weight and the dry matter was found to be 43% . DHA was found to be 6 g. i.e. 1.2 g/L . Thus, it was clear that the organism was unable to utilize disaccharides.

### Example 2 - Fermentation with lactose

### (A) Start of seed culture - Schizochytrium limacinum ATCC MYA 1381

100 ml of seed medium was prepared in 250 ml conical flask containing 100 ml water, 0.2 g dextrose, 2g of lactose, 0.25 g yeast extract, 4 g soy flour and 3.2 g sea salt. The pH was adjusted to 7.5 using 1 N sodium hydroxide and was autoclaved. One loop full of Schizochitrium limacinum ATCC MYA 1381 was transferred to the flask and was incubated at 22°C at 220 RPM for 48 hours.

750 ml of seed medium was prepared in 1000 ml conical flask containing 750ml water, 1.5 g of dextrose, 15g of lactose, 7 g starch, 2 g of Amylase, 1.125 g yeast extract, 30 g soy flour 1.125g ammonium phosphate and 24 g sea salt. The pH was adjusted to 7.5 using 1 N sodium hydroxide and was autoclaved. 7.5 ml of seed inoculum generated from 100 ml seed flask was transferred to it. The flask was kept in a shaker for 58 hours at 24°C.

### (B) Submerged batch fermentation

5000 ml of water was taken in a 5L fermenter. 20 g of dextrose, 20 g of lactose, 9 g of yeast extract, 35 g glycerol, 35 g soy flour, 14 g of ammonium phosphate and 160 g of sea salt was added to the fermenter and all the contents were dissolved. The pH of the contents in the fermenter was adjusted to 3.5 using dilute HCl. The sterilization of the media components was carried out and after that the pH was adjusted to 7.5 using 1 N sodium hydroxide. 500 ml of inoculum from example 2(A) was transferred via peristaltic pump.

The temperature was maintained at 22°C for 12 hours. The temperature was allowed to rise till 26°C and was not allowed to proceed further. The dissolved oxygen was not allowed to drop beyond 20% and the stirrer RPM was increased till 800 RPM. The pH of the medium was not allowed to drop below 5.0.

Samples were taken intermittently and analyzed for biomass and the DHA content.

The fermentation was stopped after 120 hours and the following results were obtained. The total biomass accumulated was found to be 320 g wet weight and the dry matter was found to be 43% i.e. 27.5 g/L of dry matter biomass was produced. DHA was found to be 3.5g i.e. 0.7 g/L. This also reflected on inability of the organism to utilize lactose for fementation.

### Example 3 - Semicontinuous fermentation

Seed medium was prepared in 500 ml conical flask containing 250 ml water, 5g dextrose, 2g of glycerol, 2.25 g yeast extract, 1.25 g soy flour and 1.0 g sea salt. The pH was adjusted to 5 and was autoclaved. One slant full of Schizochitrium limacinum ATCC MYA 1381 was transferred to the flask and was incubated at 26°C at 220 RPM for 48 hours.

The seed medium was transferred into 5 L inoculum preparation fermenter with 2.5 L of medium containing 2500 ml water, 50g dextrose, 20g of glycerol, 22.5 g yeast extract, 12.5 g soy flour, 1g of urea and 1.0 g sea salt. The temperature was maintained at 26°C and stirrer speed 600 RPM for 48 hrs. The dissolved oxygen was maintained above 50% with pure oxygen mix with air. The wet biomass value obtained after 48 hrs of fermentation was found to be 160g/L with a moisture content of 70%.

This inoculum was asceptically transferred to 250 L fermenter containing 20g/L of dextrose, 20g/L of lactose, 9g of yeast extract, 35g/L glycerol, 5g/L soy flour, 14 g/L of ammonium phosphate and 30 g/L of sea salt. The fermentation was carried out at 260 RPM and dissolved oxygen was maintained above 50% of saturation for the first 24 hours and then maintained above 30% for the next 48 hours with oxygen and air gas mix arrangement with PID control.

After 52 hours of fermentation the wet biomass was found to be 120g/L with a moisture content of 72.5%. 200 L of the fermented broth was transferred to another fermenter asceptically through sterilized transfer line. Fresh autoclaved medium of 200 L was replenished into the bioreactor and the fermentation was continued under similar conditions as mentioned above.

The fermenter containing the drained broth was held at 23°C and the pH was adjusted to 4.5. The dissolved oxygen was maintained at around 15% of saturation with oxygen air gas mix arrangement with PID control. The fermenter broth was harvested after 72 hours and was analyzed for lipid production. The total fat content was found to be 45g/L and DHA was found to be 18g/L.

### Example 4:

### Continuous fermentation

Seed medium was prepared in 50 ml conical flask containing 50 ml water, 0.25 g dextrose, 0.1g of glycerol, 0.125 g yeast extract, 0.12 g soy flour and 0.15 g sea salt. The pH was adjusted to 5 and was autoclaved. One loop full of Schizochitrium limacinum ATCC MYA 1381 was transferred to the flask and was incubated at 26°C at 220 RPM for 48 hours.

The seed medium was transferred into a first 5 L fermenter with 2.5 L of medium containing 2500 ml water, 50g dextrose, 20g of glycerol, 22.5 g yeast extract, 12.5 g soy flour, 1g of urea and 1.0 g sea salt. The temperature was maintained at 26°C and stirrer speed 600 RPM for 48 hrs. The dissolved oxygen was maintained above 50% with pure oxygen mix with air. After 48 hours of fermentation the wet biomass was found to be 200g/L with a moisture content of 74%. A peristaltic pump was connected to the sample line of the fermenter and was asceptically transferred to a second fermenter (7L capacity) at a flow rate of 5ml/min. Fresh sterilized media was pumped into the first fermenter asceptically at the same flow rate of 5ml/min.

The second fermenter containing the pumped broth was held at 23°C and the pH was adjusted to 4.5. The dissolved oxygen was maintained at around 15% of saturation with oxygen air gas mix arrangement with PID (Potential-Integral-Derivative controller) control. The fermenter broth was continuously harvested from second fermenter after 16 hours of start of collection of fermenter broth from first fermenter through the sample line at a flow rate of 5 ml/ min. The broth harvested from second fermenter was analyzed for lipid production. The total fat content was found to be 20g/L and DHA was found to be 6g/L. Biomass obtained was 220g/L wet weight with a Dry Mass of 34%.

### Example 5:

### Batch fermentation with higher Dissolved oxygen - ATCC 20889

### (A) Start of seed culture - Schizochytrium ATCC 20889

100 ml of seed medium was prepared in 250 ml conical flask containing 100 ml water, 0.2 g dextrose, 2g of maltodextrin, 0.25 g yeast extract, 4 g soy flour and 3.2 g sea salt. The pH was adjusted to 5.5 using 1 N sodium hydroxide and was autoclaved. One loop full of Schizochytrium ATCC 20889 was transferred to the flask and was incubated at 26°C at 220 RPM for 48 hours.

750 ml of seed medium was prepared in 1000 ml conical flask containing 750ml water, 1.5 g of dextrose, 15g of maltodextrin, 1.125 g yeast extract, 30 g soy flour 1.125g ammonium phosphate and 24 g sea salt. The pH was adjusted to 5.5 using 1 N sodium hydroxide and was autoclaved. 7.5 ml of seed inoculum generated from 100 ml seed flask was transferred to it. The flask was kept in a shaker for 48 hours at 26°C.

### (B) Submerged batch fermentation

5000 ml of water was taken in a 5L fermenter. 20 g of dextrose, 200 g of maltodextrin, 15 g of yeast extract, 35 g glycerol, 350 g soy flour, 14 g of ammonium phosphate and 160 g of sea salt was added to the fermenter and all the contents were dissolved. The pH of the contents in the fermenter was adjusted to 3.5 using dilute HCl. The sterilization of the media components was carried out and after that the pH was adjusted to 5.5 using 1 N sodium hydroxide. 500 ml of inoculum from example 5(A) was transferred via peristaltic pump.

The temperature was maintained at 26°C for 12 hours. The temperature was allowed to rise till 29°C and was not allowed to proceed further. The dissolved oxygen was not allowed to drop below 20% and the stirrer RPM was increased till 800 RPM. Further fermenter was switched to oxygen mix to maintain dissolved oxygen. The pH of the medium was not allowed to drop below 5.0. Samples were taken intermittently and analyzed for biomass and the DHA content.

The fermentation was stopped after 120 hours and the following results were obtained. The total biomass accumulated was found to be 280 g/L wet weight Per liter and the moisture was found to be 70% i.e. 84 g/L dry biomass density. The total fat accumulated was 34 g/L, DHA was found to be 12g/L.

### Example 6: Batch fermentation with no Oxygen mix - ATCC 20889

### (A) Start of seed culture - Schizochytrium ATCC 20889

100 ml of seed medium was prepared in 250 ml conical flask containing 100 ml water, 0.2 g dextrose, 2g of maltodextrin, 0.25 g yeast extract, 4 g soy flour and 3.2 g sea salt. The pH was adjusted to 5.5 using 1 N sodium hydroxide and was autoclaved. One loop full of Schizochytrium ATCC 20889 was transferred to the flask and was incubated at 26°C at 220 RPM for 48 hours.

750 ml of seed medium was prepared in 1000 ml conical flask containing 750ml water, 1.5 g of dextrose, 15g of maltodextrin, 1.125 g yeast extract, 30 g soy flour 1.125g ammonium phosphate and 24 g sea salt. The pH was adjusted to 5.5 using 1 N sodium hydroxide and was autoclaved. 7.5 ml of seed inoculum generated from 100 ml seed flask was transferred to it. The flask was kept in a shaker for 48 hours at 26°C.

### (B) Submerged batch fermentation without oxygen mix

5000 ml of water was taken in a 5L fermenter. 20 g of dextrose, 200 g of maltodextrin, 15 g of yeast extract, 35 g glycerol, 350 g soy flour, 14 g of ammonium phosphate and 160 g of sea salt was added to the fermenter and all the contents were dissolved. The pH of the contents in the fermenter was adjusted to 3.5 using dilute HCl. The sterilization of the media components was carried out and after that the pH was adjusted to 5.5 using 1 N sodium hydroxide. 500 ml of inoculum from example 6(A) was transferred via peristaltic pump.

The temperature was maintained at 26°C for 12 hours. The temperature was allowed to rise till 29°C and was not allowed to proceed further. The dissolved oxygen was maintained at 20% by increasing agitation up to 800RPM. Further when the oxygen level when dropped below, fermentation was continued and DO drop was monitored. The drop was recorded and was found to be < 5% from 48 hours till end of fermentation. The pH of the medium was not allowed to drop below 5.0. Samples were taken intermittently and analyzed for biomass and the DHA content.

The fermentation was stopped after 120 hours and the following results were obtained. The total biomass accumulated was found to be 160 g/L wet weight and the moisture was found to be 72%. Total fat was found to be 18g/L DHA was found to be 4.2 g/L.

### Example 7: Batch fermentation with higher Dissolved oxygen - ATCC 20888(A) Start of seed culture - Schizochytrium ATCC 20888

100 ml of seed medium was prepared in 250 ml conical flask containing 100 ml water, 0.2 g dextrose, 2g of maltodextrin, 0.25 g yeast extract, 4 g soy flour and 3.2 g sea salt. The pH was adjusted to 5.5 using 1 N sodium hydroxide and was autoclaved. One loop full of Schizochytrium ATCC 20888 was transferred to the flask and was incubated at 26°C at 220 RPM for 48 hours.

750 ml of seed medium was prepared in 1000 ml conical flask containing 750ml water, 1.5 g of dextrose, 15g of maltodextrin, 1.125 g yeast extract, 30 g soy flour 1.125g ammonium phosphate and 24 g sea salt. The pH was adjusted to 5.5 using 1 N sodium hydroxide and was autoclaved. 7.5 ml of seed inoculum generated from 100 ml seed flask was transferred to it. The flask was kept in a shaker for 48 hours at 26°C.

### (B) Submerged batch fermentation

5000 ml of water was taken in a 5L fermenter. 20 g of dextrose, 200 g of maltodextrin, 15 g of yeast extract, 35 g glycerol, 350 g soy flour, 14 g of ammonium phosphate and 160 g of sea salt was added to the fermenter and all the contents were dissolved. The pH of the contents in the fermenter was adjusted to 3.5 using dilute HCl. The sterilization of the media components was carried out and after that the pH was adjusted to 5.5 using 1 N sodium hydroxide. 500 ml of inoculum from example 7(A) was transferred via peristaltic pump.

The temperature was maintained at 26°C for 12 hours. The temperature was allowed to rise till 29°C and was not allowed to proceed further. The dissolved oxygen was not allowed to drop below 20% and the stirrer RPM was increased till 800 RPM. Further fermenter was switched to oxygen mix to maintain dissolved oxygen. The pH of the medium was not allowed to drop below 5.0. Samples were taken intermittently and analyzed for biomass and the DHA content.

The fermentation was stopped after 120 hours and the following results were obtained. The total biomass accumulated was found to be 265 g/L wet weight and the moisture was found to be 68%. The total fat accumulated was found to be 26 g/L. DHA was found to be 6.7g/L.

### Example 8: Batch fermentation with higher Dissolved oxygen - ATCC 20891

### (A) Start of seed culture - Schizochytrium ATCC 20891

100 ml of seed medium was prepared in 250 ml conical flask containing 100 ml water, 0.2 g dextrose, 2g of maltodextrin, 0.25 g yeast extract, 4 g soy flour and 3.2 g sea salt. The pH was adjusted to 5.5 using 1 N sodium hydroxide and was autoclaved. One loop full of Schizochytrium ATCC 20891 was transferred to the flask and was incubated at 26°C at 220 RPM for 48 hours.

750 ml of seed medium was prepared in 1000 ml conical flask containing 750ml water, 1.5 g of dextrose, 15g of maltodextrin, 1.125 g yeast extract, 30 g soy flour 1.125g ammonium phosphate and 24 g sea salt. The pH was adjusted to 5.5 using 1 N sodium hydroxide and was autoclaved. 7.5 ml of seed inoculum generated from 100 ml seed flask was transferred to it. The flask was kept in a shaker for 48 hours at 26°C.

### (B) Submerged batch fermentation

5000 ml of water was taken in a 5L fermenter. 20 g of dextrose, 200 g of maltodextrin, 15 g of yeast extract, 35 g glycerol, 350 g soy flour, 14 g of ammonium phosphate and 160 g of sea salt was added to the fermenter and all the contents were dissolved. The pH of the contents in the fermenter was adjusted to 3.5 using dilute HCl. The sterilization of the media components was carried out and after that the pH was adjusted to 5.5 using 1 N sodium hydroxide. 500 ml of inoculum from example 8(A) was transferred via peristaltic pump.

The temperature was maintained at 26°C for 12 hours. The temperature was allowed to rise till 29°C and was not allowed to proceed further. The dissolved oxygen was not allowed to drop beyond 20% and the stirrer RPM was increased till 800 RPM. Further fermenter was switched to oxygen mix to maintain dissolved oxygen. The pH of the medium was not allowed to drop below 5.0. Samples were taken intermittently and analyzed for biomass and the DHA content.

The fermentation was stopped after 120 hours and the following results were obtained. The total biomass accumulated was found to be 190 g/L wet weight and the moisture was found to be 71%. Total fat was found to be 21g/L. DHA was found to be 3.6g/L.

### Example 9 -

### Batch fermentation with higher Dissolved oxygen - ATCC 20889

### (A) Start of seed culture - Schizochytrium ATCC 20889

100 ml of seed medium was prepared in 250 ml conical flask containing 100 ml water, 0.2 g dextrose, 2g of maltodextrin, 0.25 g yeast extract, 4 g soy flour and 3.2 g sea salt. The pH was adjusted to 5.5 using 1 N sodium hydroxide and was autoclaved. One loop full of Schizochytrium ATCC 20889 was transferred to the flask and was incubated at 26°C at 220 RPM for 48 hours.

750 ml of seed medium was prepared in 1000 ml conical flask containing 750ml water, 1.5 g of dextrose, 15g of maltodextrin, 1.125 g yeast extract, 30 g soy flour 1.125g ammonium phosphate and 24 g sea salt. The pH was adjusted to 5.5 using 1 N sodium hydroxide and was autoclaved. 7.5 ml of seed inoculum generated from 100 ml seed flask was transferred to it. The flask was kept in a shaker for 48 hours at 26°C.

### (B) Submerged batch fermentation

5000 ml of water was taken in a 5L fermenter. 350 g of dextrose, , 8 g of yeast extract, 20 g soy flour, 2.5 g of ammonium phosphate and 160 g of sea salt was added to the fermenter and all the contents were dissolved. The pH of the contents in the fermenter was adjusted to 3.5 using dilute HCl. The sterilization of the media components was carried out and after that the pH was adjusted to 5.5 using 1 N sodium hydroxide. 500 ml of inoculum from example 9(A) was transferred via peristaltic pump.

The temperature was maintained at 26°C for 12 hours. The temperature was allowed to rise till 29°C and was not allowed to proceed further. The dissolved oxygen was not allowed to drop below 20% and the stirrer RPM was increased till 800 RPM. Further fermenter was switched to oxygen mix to maintain dissolved oxygen. The pH of the medium was not allowed to drop below 5.0.

Samples were taken intermittently and analyzed for biomass and the DHA content.

The fermentation was stopped after 120 hours and the following results were obtained. The total biomass accumulated was found to be 295 g/L wet weight Per liter and the moisture was found to be 68% i.e. 94 g/L dry biomass density. The total fat accumulated was 37 g/L, DHA was found to be 13.2g/L.

By varying the quantity of dextrose addition around 250 to 400 g/liter and period of fermentation around 120 to 140 hours, and reasonably varying other ingredients of the medium within limits that would be done by a person skilled in the art, performance given in Table 1 and 2 was achieved for batch fermentation of about 350 g dextrose by maintaining dissolved oxygen level of 30% or more throughout the fermentation.

**Table 1**

| **Batch fermentation results for production of DHA** | | | | | |
|---|---|---|---|---|---|
| **S. No.** | **Age (hour)** | **Biomass (g/Liter) on dry matter basis** | **DHA % of dry biomass** | **DHA (g/Liter of fermentation medium)** | **DHA (g/Liter/hour)** |
| 1 | 120 | 84 | 14.2 | 11.9 | 0.09917 |
| 2 | 100 | 83 | 12.2 | 10.1 | 0.10100 |
| 3 | 140 | 89 | 14.6 | 12.9 | 0.09214 |
| 4 | 125 | 76 | 13.6 | 10.3 | 0.08240 |
| 5 | 115 | 78 | 12.9 | 10 | 0.08696 |
| 6 | 120 | 87 | 14.6 | 12.7 | 0.10583 |
| 7 | 125 | 84 | 14 | 11.7 | 0.09360 |
| 8 | 100 | 85 | 12.9 | 10.9 | 0.10900 |
| 9 | 115 | 79 | 14.2 | 11.2 | 0.09739 |
| 10 | 135 | 86 | 15.2 | 13 | 0.09630 |
| Mean | 119.5 | 83.1 | 13.84 | 11.47 | 0.09638 |
| Standard Devation | 4.11 | 1.32 | 0.29 | 0.36 | 0.00256 |

**Table 2**

| | | | | | |
|---|---|---|---|---|---|
| | | | | | |

| **Batch fermentation results for production of DHA (Scaled up to 500 liter)** | | | | | |
|---|---|---|---|---|---|
| **S. No.** | **Age (hour)** | **Biomass (g/Liter) on dry matter basis** | **DHA % of dry biomass** | **DHA (g/Liter of fermentation medium)** | **DHA (g/Liter/hour)** |
| 1 | 100 | 76 | 11.2 | 8.5 | 0.08500 |
| 2 | 115 | 79 | 14.2 | 10.1 | 0.08783 |
| 3 | 120 | 84 | 14.3 | 12 | 0.10000 |
| 4 | 125 | 73 | 16.8 | 12.2 | 0.09760 |
| 5 | 130 | 76 | 14.4 | 10.9 | 0.08385 |
| Mean | 118 | 77.6 | 14.18 | 10.74 | 0.09085 |
| Standard Deviation | 5.15 | 1.86 | 0.89 | 0.68 | 0.00333 |

## Claims

1. A process of producing polyunsaturated fatty acids from Thraustochytriales at high availability of oxygen comprising: obtaining more than 2 g Docosahexaeonic acid (DHA) per liter of fermentation medium in submerged fermentation by maintaining dissolved oxygen at more than 8% saturation throughout the fermentation; wherein the process comprises steps of initially increasing the agitation tip speed of a stirrer used to stir the medium up to a tip speed of 3.2 m/ sec, and of switching to gas mix mode where pure oxygen is mixed with air and sparged through the fermenter medium to prevent dissolved oxygen level dropping below a targeted level of around 15% or 20% or 30% or 50% of saturation or more in an advanced stage of fermentation in addition to agitation at said tip speed.

2. The process of claim 1, comprising a batch fermentation, a semicontinuous fermentation or a continuous fermentation.

3. The process of claim 2, comprising a batch fermentation, wherein yields of DHA higher than 3 g/Liter, more preferably higher than 10g/Liter are obtained by maintaining at least 20% dissolved oxygen level in the medium.

4. The process of claim 1, wherein air is mixed with pure oxygen to carry 1 to 60% oxygen.

5. The process of claim 4, wherein a biomass density of more than 100g/L dry cell weight with DHA accumulation of 2 g/Lit or more is obtained.

6. The process of claim 4, comprising a batch fermentation, wherein reducing sugars up to 400 g/Liter are added to the fermentation medium at the beginning of the batch fermentation.

7. The process of claim 2 or claim 6, comprising a batch fermentation, wherein inorganic or urea nitrogen addition is supplemented with addition of organic nitrogen derived from amino groups containing compounds.

8. The process of claim 7, comprising a batch fermentation, wherein said organic nitrogen containing peptide bonds is protein rich or peptide rich source or amino acids.

9. The process of claim 8, comprising a batch fermentation, wherein said protein rich source comprises defatted soy-flour.

10. The process of claim 1 or claim 7 or claim 9, comprising addition of an anti-foaming agent.

11. The process of claim 2, comprising a semi-continuous process, further comprising steps of:
a. fermenting a batch upto a point where the organism is in actively dividing state in a log phase of growth in first fermenter,
b. retaining a portion of the fermenting medium in the first fermenter in a volume enough to provide an actively dividing inoculum to the next batch, aseptically transferring the rest of the actively fermenting medium to a second reactor,
c. restoring the volume of the first reactor by adding sterile fermentation medium to the same and subjecting the same to the step (a) and repeating this step for desired number of times, or optionally (i) to allow the balance volume to step (d) or (ii) to restore the volume of the first reactor by adding sterile fermentation medium to the same and allowing it to complete further growth and lipid production which is to be harvested to end the series of semi-continuous production,
d. providing conditions to the medium in the second fermenter to divide up to their potential that is limited by the nutrient availability and to produce DHA,
e. recovering the DHA .

12. The process of claim 2, comprising a continuous process, further comprising the steps of:
a. fermenting a batch upto a point where the organism is in actively dividing state in a log phase of growth in first fermenter,
b. taking precautions to avoid entry of contaminants, starting addition of sterile medium to the first fermenter and allowing aseptic drain off or removal of same volume of fermenting medium to a second fermenter at such a rate that the contents of the first reactor remain always at log phase of growth,
c. providing conditions to the medium in the second fermenter to divide upto their potential that is limited by the nutrient availability and to produce DHA,
d. after the second fermenter fills up to the capacity, providing for aseptic drain off or removal of medium excess of the capacity from the second fermenter,
e. recovering DHA immediately or after providing a further interval of time.

13. The process of claim 1, wherein the microorganism used for fermentation is a Thraustochytrid or Schizochytrium.

14. The process of claim 11, wherein said anti-foaming agent is a silicone antifoam.

## Patentansprüche

1. Verfahren zum Erzeugen von mehrfach ungesättigten Fettsäuren aus Thraustochytriales bei hoher Verfügbarkeit von Sauerstoff, das folgende Schritte umfasst: Erhalten von mehr als 2 g Docosahexaensäure (DHA) pro Liter Fermentierungsmedium bei einer Tauchfermentierung durch Beibehalten von gelöstem Sauerstoff auf mehr als 8 % Sättigung während der gesamten Fermentierung; wobei das Verfahren die Schritte des anfänglichen Erhöhens der Rührbewegungs-Höchstgeschwindigkeit eines Rührers, der zum Verrühren des Mediums verwendet wird, bis zu einer Höchstgeschwindigkeit von 3,2 m/sec und des Schaltens auf einen Gasmischmodus beinhaltet, in dem reiner Sauerstoff mit Luft vermischt und durch das Fermentierungsmedium geblasen wird, so dass verhindert wird, dass der Wert des gelösten Sauerstoffs einen Soll-Wert von etwa 15 % oder 20 % oder 30 % oder 50 % Sättigung oder mehr in einem fortgeschrittenen Stadium der Fermentierung neben einer Rührbewegung bei der Höchstgeschwindigkeit unterschreitet.

2. Verfahren nach Anspruch 1, wobei das Verfahren eine chargenweise Fermentierung, eine halbkontinuierliche Fermentierung oder eine kontinuierliche Fermentierung beinhaltet.

3. Verfahren nach Anspruch 2, wobei das Verfahren eine chargenweise Fermentierung beinhaltet, wobei DHA-Erträge von mehr als 3 g/l, und bevorzugt mehr als 10 g/l, durch Beibehalten eines Werts von zumindest 20 % gelöstem Sauerstoff in dem Medium erhalten werden.

4. Verfahren nach Anspruch 1, wobei Luft mit reinem Sauerstoff vermischt wird, so dass sie 1 bis 60 % Sauerstoff trägt.

5. Verfahren nach Anspruch 4, wobei eine Biomassedichte von mehr als 100 g/l Trockenzellengewicht mit einer DHA-Akkumulation von 2 g/l oder mehr erhalten wird.

6. Verfahren nach Anspruch 4, wobei das Verfahren eine chargenweise Fermentierung beinhaltet, wobei dem Fermentierungsmedium reduzierende Zucker von bis zu 400 g/l zu Beginn der chargenweisen Fermentierung hinzugefügt werden.

7. Verfahren nach Anspruch 2 oder Anspruch 6, wobei das Verfahren eine chargenweise Fermentierung beinhaltet, wobei eine anorganische Addition oder Hamstoff-Stickstoff-Addition unter Zugabe von organischem Stickstoff ergänzt wird, der aus aminogruppenhaltigen Verbindungen gewonnen wird.

8. Verfahren nach Anspruch 7, wobei das Verfahren eine chargenweise Fermentierung beinhaltet, wobei der organische Stickstoff, der Peptidbindungen enthält, eine proteinreiche oder peptidreiche Quelle oder Aminosäuren sind.

9. Verfahren nach Anspruch 8, wobei das Verfahren eine chargenweise Fermentierung beinhaltet, wobei die proteinreiche Quelle entfettetes Sojamehl aufweist.

10. Verfahren nach Anspruch 1 oder Anspruch 7 oder Anspruch 9, wobei das Verfahren die Zugabe von einem Antischaummittel beinhaltet.

11. Verfahren nach Anspruch 2, wobei das Verfahren ein halbkontinuierliches Verfahren beinhaltet, das ferner folgende Schritte beinhaltet:
a. Fermentieren einer Charge bis zu einem Punkt, wo der Organismus sich in einem aktiven Teilungszustand in einer Log-Phase des Wachstums in einem ersten Fermenter befindet,
b. Zurückhalten eines Teils des Fermentierungsmediums in dem ersten Fermenter in einem Volumen, das zum Bereitstellen eines aktiven Teilungsinokulum für die nächste Charge ausreicht, aseptisches Übertragen des verbleibenden aktiven Fermentierungsmediums auf einen zweiten Reaktor,
c. Wiederherstellen des Volumens des ersten Reaktors durch Zugabe von einem sterilen Fermentierungsmedium in denselben und Unterziehen desselbigen von Schritt (a) und Wiederholen dieses Schritts für eine gewünschte Anzahl von Malen, oder optional (i) Überlassen des Ausgleichsvolumens an Schritt (d) oder (ii) Wiederherstellen des Volumens des ersten Reaktors durch Zugabe des sterilen Fermentierungsmediums in denselben und dasselbe ein weiteres Wachstum und eine weitere Lipidproduktion vollenden lassen, die zum Beenden der Reihe der halbkontinuierlichen Produktion entnommen werden soll,
d. Schaffen von Bedingungen für das Medium in dem zweiten Fermenter, so dass dessen Teilungspotential ausgeschöpft werden kann, das durch die Nährstoffverfügbarkeit begrenzt ist, und DHA erzeugt werden kann.
e. Wiedergewinnung der DHA.

12. Verfahren nach Anspruch 1, das wobei das Verfahren ein kontinuierliches Verfahren beinhaltet, das weiterhin folgende Schritte beinhaltet:
a. Fermentieren einer Charge bis zu einem Punkt, wo der Organismus sich in einem aktiven Teilungszustand in einer Log-Phase des Wachstums in einem ersten Fermenter befindet,
b. Ergreifen von Vorsichtsmaßnahmen zum Verhindern eines Eindringens von Verschmutzungen, Beginnen mit der Zugabe des sterilen Mediums in den ersten Fermenter und Zulassen eines aspetischen Ablaufs oder einer Entnahme des gleichen Volumens des Fermentierungsmediums in einen zweiten Fermenter bei einer Rate, bei der der Inhalt des ersten Reaktors stets in der Log-Phase des Wachstums verbleibt,
c. Schaffen von Bedingungen für das Medium in dem zweiten Fermenter, so dass dessen Teilungspotential ausgeschöpft werden kann, das durch die Nährstoffverfügbarkeit begrenzt ist, und DHA erzeugt werden kann.
d. nachdem sich der zweite Fermenter bis zur maximalen Füllmenge gefüllt hat, Ermöglichen eines aseptischen Ablaufs oder einer Entnahme des Mediums, das die maximale Füllmenge überschritten hat, aus dem zweiten Fermenter,
e. Wiederherstellen von DHA unmittelbar oder nach Vorsehen eines weiteren Zeitintervalls.

13. Verfahren nach Anspruch 1, wobei der Mikroorganismus, der für die Fermentierung verwendet wird, ein Thraustochytrid oder Schizochytrium ist.

14. Verfahren nach Anspruch 11, wobei das Antischaummittel ein Silikon-Antischaummittel ist.

## Revendications

1. Procédé de production d'acides gras polyinsaturés à partir de thraustochytriales à haute disponibilité en oxygène comprenant : l'obtention de plus de 2 g d'acides docosahexaènoïque (DHA) par litre de milieu de fermentation dans une fermentation submergée en maintenant l'oxygène dissous à une saturation de plus de 8 % tout au long de la fermentation ; dans lequel le procédé comprend les étapes d'augmentation initiale de la vitesse périphérique d'agitation d'un agitateur utilisé pour agiter le milieu jusqu'à une vitesse périphérique de 3,2 m/seconde,
et de passage à un mode de mélange de gaz où de l'oxygène pur est mélangé avec de l'air et dispersé à travers le milieu du fermenteur pour empêcher que le taux d'oxygène dissous chute en dessous d'un taux cible d'environ 15 % ou 20 % ou 30 % ou 50 % de saturation ou plus à un stade avancé de fermentation en plus d'une agitation à ladite vitesse périphérique.

2. Procédé selon la revendication 1, comprenant une fermentation discontinue, une fermentation semi-continue ou une fermentation continue.

3. Procédé selon la revendication 2, comprenant une fermentation discontinue, dans lequel des rendements en DHA supérieurs à 3 g/litre, de manière davantage préférée supérieurs à 10 g/litre sont obtenus en maintenant un taux d'oxygène dissous d'au moins 20 % dans le milieu.

4. Procédé selon la revendication 1, dans lequel de l'air est mélangé avec de l'oxygène pur pour porter 1 à 60 % d'oxygène.

5. Procédé selon la revendication 4, dans lequel une masse volumique de biomasse de plus de 100 g/l de poids de cellules sèches avec une accumulation de DHA de 2 g/l ou plus est obtenue.

6. Procédé selon la revendication 4, comprenant une fermentation discontinue, dans lequel des sucres réducteurs jusqu'à 400 g/litre sont ajoutés au milieu de fermentation au début de la fermentation discontinue.

7. Procédé selon la revendication 2 ou la revendication 6, comprenant une fermentation discontinue, dans lequel l'ajout d'azote inorganique ou d'urée est complété avec l'ajout d'azote organique dérivé de composés contenant des groupes aminés.

8. Procédé selon la revendication 7, comprenant une fermentation discontinue, dans lequel ledit azote organique contenant des liaisons peptidiques est une source riche en protéines ou riche en peptides ou des acides aminés.

9. Procédé selon la revendication 8, comprenant une fermentation discontinue, dans lequel ladite source riche en protéines comprend une farine de soja dégraissée.

10. Procédé selon la revendication 1 ou la revendication 7 ou la revendication 9, comprenant l'ajout d'un agent antimousse.

11. Procédé selon la revendication 2, comprenant un procédé semi-continu, comprenant en outre les étapes suivantes :
a. la fermentation d'un lot jusqu'à un point où l'organisme est dans un état de division active dans une phase logarithmique de croissance dans un premier fermenteur,
b. la conservation d'une partie du milieu de fermentation dans le premier fermenteur dans un volume suffisant pour fournir un inoculum en division active au prochain lot, le transfert de manière aseptique du reste du milieu en fermentation active vers un second réacteur,
c. la restauration du volume du premier réacteur par l'ajout d'un milieu de fermentation stérile à celui-ci et la soumission de celui-ci à l'étape (a) et la répétition de cette étape un nombre souhaité de fois, ou facultativement (i) laisser le volume d'équilibre à l'étape (d) ou (ii) restaurer le volume du premier réacteur par l'ajout d'un milieu fermentation stérile à celui-ci et lui permettre d'achever une croissance et une production de lipide supplémentaires qui doit être collectée pour terminer la série de production semi-continue,
d. la fourniture de conditions au milieu dans le second fermenteur pour diviser jusqu'à leur potentiel qui est limité par la disponibilité en nutriments et pour produire du DHA,
e. la récupération du DHA.

12. Procédé selon la revendication 2, comprenant un procédé continu, comprenant en outre les étapes suivantes :
a. la fermentation d'un lot jusqu'à un point où l'organisme est dans un état de division active dans une phase logarithmique de croissance dans un premier fermenteur,
b. le fait de prendre soin d'éviter l'entrée de contaminants, l'initiation de l'ajout d'un milieu stérile au premier fermenteur et le fait de vider ou de retirer de manière aseptique le même volume de milieu de fermentation vers un second fermenteur à un débit tel que le contenu du premier réacteur reste toujours dans une phase logarithmique de croissance,
c. la fourniture de conditions au milieu dans le second fermenteur pour diviser jusqu'à leur potentiel qui est limité par la disponibilité en nutriments et pour produire du DHA,
d. après le remplissage du second fermenteur jusqu'à la capacité, le fait de vider ou de retirer de manière aseptique le milieu en excès de la capacité à partir du second fermenteur,
e. la récupération du DHA immédiatement ou après la fourniture d'un intervalle de temps supplémentaire.

13. Procédé selon la revendication 1, dans lequel le micro-organisme utilisé pour la fermentation est un thraustochytride ou une schizochytrium.

14. Procédé selon la revendication 11, dans lequel ledit agent antimousse est un antimousse à base de silicone.
